(19) Europäisches Patentamt
European Patent Office
Office européen des brevets

(11) Veröffentlichungsnummer : **0 527 112 A2**

# (12) EUROPÄISCHE PATENTANMELDUNG

(21) Anmeldenummer : **92810595.6**

(22) Anmeldetag : **04.08.92**

(51) Int. Cl.$^5$ : **C07C 243/28,** A01N 51/00, C07C 281/06, C07C 281/02, C07C 243/38, C07C 337/06, C07D 295/32, C07D 213/86, C07C 259/06

(30) Priorität : **07.08.91 CH 2336/91**

(43) Veröffentlichungstag der Anmeldung : **10.02.93 Patentblatt 93/06**

(84) Benannte Vertragsstaaten : **AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Maienfisch, Peter, Dr.**
**Aegertenstrasse 21**
**CH-4118 Rodersdorf (CH)**
Erfinder : **Böger, Manfred**
**Wilhelm Glockstrasse 14**
**W-7858 Weil am Rhein 5 (DE)**
Erfinder : **Pitterna, Thomas, Dr.**
**Frobenstrasse 78**
**CH-4053 Basel (CH)**

## (54) Buttersäureamide als Schadlingsbekämpfungmittel.

(57) Verbindungen der Formel

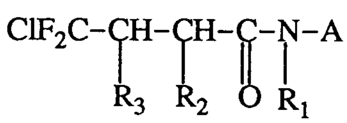

(I),

worin
A einen unsubstituierten oder substituierten, über ein Ringstickstoffatom gebundenen, aromatischen oder nicht-aromatischen, mono- oder bicyclischen, stickstoffhaltigen Heterocyclus oder eine Gruppe $-OR_4$, $-N(R_4)R_5$, $-N(R_5)COR_4$, $-N(R_5)COOR_4$, $-N(R_5)CON(R_4)R_6$ oder $-N(R_5)CSN(R_4)R_6$ ;
$R_1$ Wasserstoff, $C_1$-$C_6$-Alkyl, $C_1$-$C_6$-Halogenalkyl oder Benzyl ;
$R_2$ und $R_3$ unabhängig voneinander Wasserstoff oder $C_1$-$C_4$-Alkyl ;
$R_4$ Wasserstoff, eine unsubstituierte oder substituierte $C_1$-$C_{20}$-Alkyl-, $C_3$-$C_{10}$-Alkenyl-, $C_3$-$C_{10}$-Alkinyl-, $C_3$-$C_7$-Cycloalkyl- oder Aryl-Gruppe oder einen unsubstituierten oder substituierten, aromatischen oder nicht-aromatischen, über ein Ringkohlenstoffatom gebundenen, mono- oder bicyclischen, Heterocyclus ; und
$R_5$ und $R_5$ unabhängig voneinander Wasserstoff, $C_1$-$C_6$-Alkyl oder Phenyl bedeuten, in freier Form oder in Salzform, können als agrochemische Wirkstoffe verwendet werden und sind in an sich bekannter Weise herstellbar.

EP 0 527 112 A2

Die vorliegende Erfindung betrifft neue Derivate von 4-Chlor-4,4-difluor-buttersäureamiden, Verfahren zu ihrer Herstellung, Schädlingsbekämpfungsmittel, welche diese Verbindungen enthalten, sowie ihre Verwendung bei der Kontrolle von Schädlingen.

Die erfindungsgemässen 4-Chlor-4,4-difluor-buttersäureamid-Derivate entsprechen der Formel

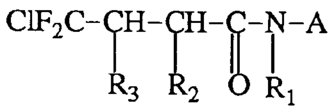

(I),

worin

A einen unsubstituierten oder substituierten, über ein Ringstickstoffatom gebundenen, aromatischen oder nicht-aromatischen, mono- oder bicyclischen, stickstoffhaltigen Heterocyclus oder eine Gruppe $-OR_4$, $-N(R_4)R_5$, $-N(R_5)COR_4$, $-N(R_5)COOR_4$, $-N(R_5)CON(R_4)R_6$ oder $-N(R_5)CSN(R_4)R_6$;

$R_1$ Wasserstoff, $C_1$ -$C_6$-Alkyl, $C_1$-$C_6$-Halogenalkyl oder Benzyl;

$R_2$ und $R_3$ unabhängig voneinander Wasserstoff oder $C_1$-$C_4$-Alkyl;

$R_4$ Wasserstoff, eine unsubstituierte oder substituierte $C_1$-$C_{20}$-Alkyl-, $C_3$-$C_{10}$-Alkenyl-, $C_3$-$C_{10}$-Alkinyl-, $C_3$-$C_7$-Cycloalkyl- oder Aryl-Gruppe oder einen unsubstituierten oder substituierten, aromatischen oder nicht-aromatischen, über ein Ringkohlenstoffatom gebundenen, mono- oder bicyclischen, Heterocyclus; und

$R_5$ und $R_5$ unabhängig voneinander Wasserstoff, $C_1$-$C_6$-Alkyl oder Phenyl bedeuten.

In EP-A-0 413 666 und US-4,950,666 werden 4-Chlor-4,4-difluor-buttersäure-Derivate als Wirkstoffe in Schädlingsbekämpfungsmitteln vorgeschlagen. Die biologischen Eigenschaften der in diesen Publikationen beschriebenen Verbindungen vermögen auf dem Gebiet der Schädlingsbekämpfung jedoch nicht voll zu befriedigen, weshalb das Bedürfnis besteht, weitere Verbindungen mit schädlingsbekämpfenden Eigenschaften zur Verfügung zu stellen, wobei diese Aufgabe erfindungsgemäss durch die Bereitstellung der vorliegenden Verbindungen I gelöst wird.

Die erfindungsgemässen Verbindungen I schliessen auch, insbesondere agrochemisch verträgliche, Säureadditionssalze ein. Beispiele geeigneter (anorganischer oder organischer) Säuren sind Salzsäure, Bromwasserstoffsäure, Schwefelsäure, Phosphorsäure, Salpetersäure, Säuren mit gleichem Zentralatom und höherer oder niedrigerer Oxidationsstufe, wie Perchlorsäure, salpetrige Säure oder phosphorige Säure, Essigsäure und Bernsteinsäure.

Die Variable A umfasst insbesondere die folgenden Gruppen als besondere Ausführungsformen der Erfindung: $-OR_4$, $-N(H)R_4$, $-N(CH_3)R_4$, $-N(C_6H_5)R_4$, $-N(H)COR_4$, $-N(CH_3)COR_4$, $-N(H)COOR_4$, $-N(H)CON(H)R_4$, $-N(H)CON(C_6H_5)R_4$, $-N(H)CSN(H)R_4$, Pyrrolidin-1-yl, Piperidin-1-yl, Perhydroazepin-1-yl, Morpholin-4-yl, 4-Methylpiperazin -1-yl, Triazol-N-yl und Phthalimid-N-yl.

Die Variable $R_4$ steht im Rahmen der vorliegenden Erfindung vorzugsweise für $C_1$-$C_{10}$-Alkyl; $C_3$-$C_7$-Cycloalkyl; $C_3$-$C_{10}$-Alkenyl; $C_3$-$C_{10}$-Alkinyl; Aryl; $C_3$-$C_{10}$-Halogenalkenyl; $C_3$-$C_{10}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyano, Benzoyl, Halogenbenzoyl, Phenoxy, Halogenphenoxy, $C_1$-$C_4$-Alkylphenoxy, $C_1$-$C_4$-Halogenalkylphenoxy, $C_1$-$C_4$-Alkylamino, Di-$C_1$-$C_4$-alkylamino, Anilino, N-$C_1$-$C_4$-Alkylanilino, N-$C_1$-$C_6$-Alkylcarbonylanilino, Phenylthio oder Halogenphenylthio substituiertes Aryl; einen über ein Ringkohlenstoffatom gebundenen, aromatischen oder nicht-aromatischen, mono- oder bicyclischen, Heterocyclus, welcher unsubstituiert oder durch Halogen, $C_1$-$C_4$-Alkyl, Nitro, $C_1$-$C_4$-Halogenalkyl, $C_1$-$C_4$-Halogenalkoxy, $C_1$-$C_4$-Alkoxy, $C_1$-$C_4$-Alkylthio oder Cyclopropyl substituiert sein kann; oder durch Hydroxy, Halogen, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy $C_3$-$C_7$-Cycloalkyl, Aryl, Aryloxy, Arylthio, Arylsulfonyl, Arylsulfinyl, Arylsulfonyloxy, Arylcarbonyl oder Pyridyl substituiertes $C_1$-$C_{20}$-Alkyl, wobei die Aryl- und Pyridylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro, Cyano, Phenoxy, Halogenphenoxy, Phenylthio oder Halogenphenylthio substituiert sein können; $R_4$ kann ferner auch Wasserstoff, durch Hydroxy substituiertes Aryl oder durch Cyano substituiertes $C_1$-$C_{20}$-Alkyl sein.

Die vor- und nachstehend verwendeten Allgemeinbegriffe haben, sofern nicht abweichend definiert, die nachfolgend aufgeführten Bedeutungen.

Bei den als Substituenten in Betracht kommenden Halogenatomen handelt es sich sowohl um Fluor und Chlor als auch um Brom und Iod, wobei Fluor, Chlor und Brom bevorzugt sind. Halogen ist dabei als selbständiger Substituent oder als Teil eines Substituenten zu verstehen, wie in Halogenalkyl, Halogenalkylthio, Halogenalkoxy, Halogenphenylthio, Halogenalkenyl, Halogenalkinyl, Halogenbenzoyl, Halogenalkylphenoxy

oder Halogenphenoxy.

Kohlenstoffhaltige Gruppen und Verbindungen enthalten, sofern nicht abweichend definiert, jeweils vorzugsweise 1 bis und mit 4, insbesondere 1 oder 2, Kohlenstoffatome.

$C_3$-$C_7$-Cycloalkyl ist Cyclopropyl, Cyclobutyl, Cyclopentyl, Cyclohexyl oder Cycloheptyl.

Alkyl - als Gruppe per se sowie als Strukturelement von anderen Gruppen und Verbindungen, wie von Alkoxy, Alkylthio, Alkylphenoxy, (Di-)Alkylamino, N-Alkylanilino, N-Alkylcarbonylanilino, Alkoxyalkoxy, Alkylsulfinyl, Alkylsulfonyl; Alkylsulfonyloxy, Halogenalkyl, Halogenalkoxy, Halogenalkylthio, Halogenalkylphenoxy, Alkylcarbonyl, Alkylcarbonyloxy und Alkoxycarbonyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig oder verzweigt. Als Beispiele für Alkyl seien Methyl, Aethyl, Propyl, Isopropyl, Butyl, Isobutyl, sek.-Butyl und tert.-Butyl sowie Pentyl, Hexyl, Octyl, Decyl, Dodecyl und jeweils deren Isomere genannt. Als Beispiele für Alkoxy seien Methoxy, Aethoxy, Propoxy und Isopropoxy sowie Butoxy und dessen Isomere genannt. Alkylthio ist Methylthio, Aethylthio, Propylthio oder Isopropylthio oder Butylthio oder ein Isomeres davon. In Alkylphenoxy trägt die Phenoxygruppe einen, zwei oder drei Alkylreste. In Alkoxyalkoxy ist eine an den restlichen Teil der Verbindung I gebundene Alkoxygruppe durch eine weitere Alkoxygruppe substituiert; Beispiele für Alkoxyalkoxy sind Methoxymethoxy, Methoxyäthoxy, Aethoxyäthoxy, Aethoxymethoxy, Propoxymethoxy, Methoxypropoxy, Butoxymethoxy und Propoxyäthoxy. Beispiele für Alkoxycarbonyl sind Methoxycarbonyl, Aethoxycarbonyl, Propoxycarbonyl, Isopropoxycarbonyl und Butoxycarbonyl. Alkylcarbonyl steht beispielsweise für Acetyl, Propionyl, Butyryl oder Valeryl oder gegebenenfalls Isomere davon. Alkylcarbonyloxy steht zum Beispiel für Acetoxy, Propionyloxy oder Butyryloxy.

(Halogen-)Alkenyl und (Halogen-)Alkinyl enthalten eine oder mehrere, vorzugsweise nicht mehr als drei, ungesättigte Kohlenstoff-Kohlenstoff-Bindungen. Die Doppel- oder Dreifachbindungen dieser Substituenten sind von dem restlichen Teil der Verbindung I durch mindestens ein gesättigtes Kohlenstoffatom getrennt. Beispielhaft genannt seien Allyl, Methalllyl, But-2-enyl, But-3-enyl, Propargyl, But-2-inyl und But-3-inyl.

Halogensubstituierte Gruppen, d. h. Halogenalkyl, Halogenalkylthio, Halogenalkoxy, Halogenphenylthio, Halogenalkenyl, Halogenalkinyl, Halogenbenzoyl, Halogenalkylphenoxy und Halogenphenoxy, können teilweise halogeniert oder perhalogeniert sein. Beispiele für Halogenalkyl - als Gruppe per se sowie als Strukturelement von anderen Gruppen und Verbindungen, wie von Halogenalkylthio, Halogenalkoxy oder Halogenalkylphenoxy - sind das ein- bis dreifach durch Fluor, Chlor und/oder Brom substituierte Methyl, wie $CHF_2$ oder $CF_3$; das ein- bis fünffach durch Fluor, Chlor und/oder Brom substituierte Aethyl, wie $CH_2CF_3$, $CF_2CF_3$, $CF_2CCl_3$, $CF_2Cl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl, wie $CH_2CHBrCH_2Br$, $CF_2CHFCF_3$, $CH_2CF_2CF_3$ oder $CH(CF_3)_2$; und das ein- bis neunfach durch Fluor, Chlor und/oder Brom substituierte Butyl oder eines seiner Isomeren, wie $CF(CF_3)CHFCF_3$ oder $CH_2(CF_2)_2CF_3$. Beispiele für Halogenalkenyl und Halogenalkinyl sind 2-Chlorprop-1-en-3-yl, 2,3-Dichlorprop-1-en-3-yl, 2,3-Dibromprop-1-en-3-yl und 3-Chlorprop-1-in-3-yl. Halogenbenzoyl ist im Ring halogeniert. In Halogenalkylphenoxy trägt die Phenoxygruppe einen, zwei oder drei Halogenalkyl-Substituenten.

Substituierte Alkyl-, Cycloalkyl- und Aryl-Gruppen $R_4$ können ein- oder mehrfach, vorzugsweise ein- oder zweifach, durch den gleichen oder verschiedene Substituenten substituiert sein. Substituierte Arylgruppen $R_4$, Aryl-haltige Substituenten in substituiertem Alkyl $R_4$ und Pyridyl-haltige Substituenten in substituiertem Alkyl $R_4$ können bis zu 5 gleiche oder verschiedene Substituenten tragen.

Aryl - als Gruppe per se sowie als Strukturelement von anderen Gruppen und Verbindungen, wie von Aryloxy, Arylthio, Arylsulfonyl, Arylsulfinyl, Arylsulfonyloxy und Arylcarbonyl, - steht für einen aromatischen Kohlenwasserstoffrest, vorzugsweise für Phenyl oder Naphthyl, insbesondere für Phenyl.

Beispiele für aromatische oder nicht-aromatische, über ein Ringkohlenstoffatom gebundene, mono- oder bicyclische, Heterocyclen $R_4$ sind Pyridin, Pyrazin, Pyridazin, Thiophen, Furan, Pyrimidin, Pyrrolidin, Thiazol, Thiadiazol, Oxazol, Benzothiazol, Triazin, Oxadiazol, Chinolin, Chinoxalin, Chinazolin, Isochinolin, Phthalazin, Naphthyridin, Cinnolin, Pteridin, Triazol, Piperidin und Benzoxazol. Der Heterocyclus kann Substituenten tragen, beispielsweise je bis zu drei Reste aus der Gruppe, bestehend aus Halogen, Alkyl, Halogenalkyl und Alkoxy, oder je ein oder zwei Substituenten aus der Gruppe, bestehend aus Nitro, Halogenalkoxy, Alkylthio und Cycloalkyl. Insgesamt ist die Anzahl der Substituenten am Heterocyclus in der Regel nicht grösser als vier. Vorzugsweise tragen diese Reste nicht mehr als 3 weitere Substituenten aus der Gruppe, bestehend aus Chlor, Fluor Brom, Methyl, Aethyl und Trifluormethyl. Vorzugsweise sind diese Heterocyclen $R_4$ unsubstituiert. Besonders bevorzugte Heterocyclen $R_4$ sind Pyrid-3-yl, Pyrid-4-yl, Thien-2-yl, Fur-2-yl, Pyrid-2-yl und 7-Chlorchinolin-4-yl.

Beispiele für die Grundkörper der über ein Ringstickstoffatom gebundenen, aromatischen oder nicht-aromatischen, mono- oder bicyclischen, stickstoffhaltigen Heterocyclen A sind Pyrrol, Pyrazol, Imidazol, Triazol, Pyrrolin, Imidazolin, Pyrazolin, Pyrrolidin, Piperidin, Perhydroazepin, Morpholin, Thiomorpholin, Piperazin,

Phthalimid, Indol, Indazol, Purin, Isoindol, Indolin, Carbazol und Isoindolin. Die Heterocyclen A können 1 oder 2 Substituenten tragen, ausgewählt aus der Gruppe, bestehend aus Halogen und Alkyl, sind jedoch vorzugsweise unsubstituiert. Besonders bevorzugte Heterocyclen A sind Pyrrolidin-1-yl, Piperidin-1-yl, Morpholin-4-yl, 1,2,4-Triazol-4-yl, Perhydroazepin-1-yl, 4-Methylpiperazin-1-yl und Phthalimid-N-yl.

Bevorzugte Ausführungsformen im Rahmen der Erfindung sind

(1) Eine Verbindung der Formel I, worin $R_1$ Wasserstoff ist;

(2) Eine Verbindung der Formel I, worin $R_2$ und $R_3$ Wasserstoff sind;

(3) Eine Verbindung der Formel I, worin $R_4$ Wasserstoff; $C_1$-$C_4$-Alkyl; durch Halogen oder $C_1$-$C_4$-Alkoxy substituiertes $C_1$-$C_4$-Alkyl; Phenyl; durch 1 bis 5 Halogenatome oder 1 bis 2 Substituenten, ausgewählt aus der Gruppe, bestehend aus $C_1$-$C_4$-Alkyl, $C_1$-$C_4$-Alkoxy, Cyano, $C_1$-$C_4$-Halogenalkyl und Nitro, substituiertes Phenyl; Benzyl; durch 1 bis 5 Halogenatome oder 1 bis 2 Substituenten, ausgewählt aus der Gruppe, bestehend aus $C_1$-$C_4$-Alkoxy und Nitro, substituiertes Benzyl; Thienyl; oder Furyl bedeutet;

(4) Eine Verbindung der Formel I, worin $R_1$, $R_2$ und $R_3$ Wasserstoff bedeuten, A Piperidin-1-yl, 4-$C_1$-$C_4$-Alkylpiperazin-1-yl, Phthalimid-N-yl oder eine Gruppe $-OR_4$, $-N(R_4)R_5$, $-N(R_5)COR_4$, $-N(R_5)COOR_4$, $-N(R_5)CON(R_4)R_6$ oder $-N(R_5)CSN(R_4)R_5$ ist, $R_4$ $C_1$-$C_4$-Alkyl; Halogen-$C_1$-$C_4$-alkyl mit 1 bis und mit 3 Halogenatomen; Phenyl; durch 1 bis 5 Halogenatome oder 1 bis 2 Substituenten, ausgewählt aus der Gruppe, bestehend aus $C_1$-$C_4$-Alkoxy und Nitro, substituiertes Phenyl; Benzyl; durch 1 bis 5 Halogenatome oder 1 bis 2 Substituenten, ausgewählt aus der Gruppe, bestehend aus $C_1$-$C_4$-Alkoxy und Nitro, substituiertes Benzyl; Pyridyl; Thienyl; oder Furyl bedeutet; $R_5$ Wasserstoff, $C_1$-$C_4$-Alkyl oder Phenyl ist und $R_5$ Wasserstoff ist;

(5) Eine Verbindung der Formel I, worin A $-OR_4$, $-N(R_4)R_5$, $-N(R_5)COR_4$ oder $-N(R_5)COOR_4$ ist und $R_5$ Wasserstoff, Methyl oder Phenyl ist;

(6) Eine Verbindung der Formel I, worin $R_1$, $R_2$ und $R_3$ Wasserstoff bedeuten, A eine Gruppe $-N(R_4)R_5$, $-N(R_5)COR_4$ oder $-N(R_5)COOR_4$ ist, $R_4$ $C_1$-$C_4$-Alkyl, Halogen-$C_1$-$C_4$-alkyl mit 1 bis und mit 3 Halogenatomen, Phenyl, durch 1 bis 5 Halogenatome substituiertes Phenyl oder durch $C_1$-$C_4$-Alkoxy substituiertes Benzyl bedeutet und $R_5$ Wasserstoff oder Phenyl ist.

Besonders bevorzugt sind im Rahmen der Erfindung die in den Beispielen H1 bis H5 genannten Verbindungen der Formel I.

Namentlich bevorzugt sind im Rahmen der Erfindung

(a) 4-Chlor-4,4-difluor-N-methoxy-buttersäureamid,

(b) 4-Chlor-4,4-difluor-N-(äthoxycarbonylamino)-buttersäureamid,

(c) 4-Chlor-4,4-difluor-N-(phenylamino)-buttersäureamid,

(d) 4-Chlor-4,4-difluor-N-(phenylcarbonylamino)-buttersäureamid,

(e) 4-Chlor-4,4-difluor-N-(phenylaminocarbonylamino)-buttersäureamid,

(f) 4-Chlor-4,4-difluor-N-(N,N-dimethylamino)-buttersäureamid,

(g) 4-Chlor-4,4-difluor-N-(methylcarbonylamino)-buttersäureamid,

(h) 4-Chlor-4,4-difluor-N-(fur-2-ylcarbonylamino)-buttersäureamid,

(i) 4-Chlor-4,4-difluor-N-(4-methoxybenzyloxycarbonylamino)-buttersäureamid,

(j) 4-Chlor-4,4-difluor-N-(thien-2-ylcarbonylamino)-buttersäureamid,

(k) 4-Chlor-4,4-difluor-N-(tert.-butoxycarbonylamino)-buttersäureamid und

(l) 4-Chlor-4,4-difluor-N-(N-methyl-N-phenyl-amino)-buttersäureamid.

Als weiterer Gegenstand der Erfindung ist das Verfahren zur Herstellung der Verbindungen der Formel I, in freier Form oder in Salzform, beispielsweise dadurch gekennzeichnet, dass man

a) eine Verbindung der Formel

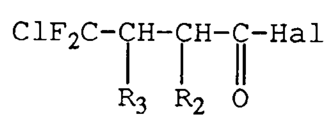

(II),

worin $R_2$ und $R_3$ die für die Formel I angegebenen Bedeutungen haben und Hal für Halogen, vorzugsweise Chlor oder Brom, steht, in Gegenwart einer Base mit einer Verbindung der Formel

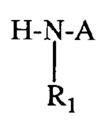

(III),

worin A und $R_1$ die für die Formel I angegebenen Bedeutungen haben, oder einem Salz davon umsetzt oder

b) eine Verbindung der Formel

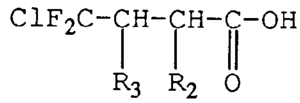

(IV),

worin $R_2$ und $R_3$ die für die Formel I angegebenen Bedeutungen haben, in Gegenwart eines Kondensationsmittels mit einer Verbindung der Formel III oder einem Salz davon umsetzt oder

c) eine Verbindung der Formel

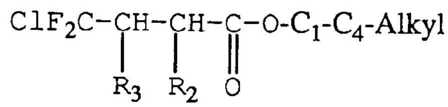

(V),

worin $R_2$ und $R_3$ die für die Formel I angegebenen Bedeutungen haben, mit einer Verbindung der Formel III oder einem Salz davon umsetzt

und jeweils, wenn erwünscht, eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

Geeignete Basen für die <u>Verfahrensvariante a)</u> sind z. B. organische Basen, beispielsweise Pyridin, 4-Dimethylaminopyridin, Lutidin, Collidin, Trialkylamine, N,N-Dialkylanilin oder bicyclische nicht-nucleophile Basen, wie 1,4-Diazabicyclo[2.2.2]octan (DABCO), 1,5-Diazabicyclo[4.3.0]non-5-en (DBN) und 1,8-Diazabicyclo[5.4.0]undec-7-en (DBU). Die Reaktion wird im allgemeinen bei Temperaturen von -30 bis +70°C, vorzugsweise von - 10 bis +50°C, durchgeführt. Man arbeitet dabei zweckmässigerweise in Gegenwart eines inerten Lösungsmittels oder Lösungsmittelgemisches. Es eignen sich hierfür beispielsweise aliphatische und aromatische Kohlenwasserstoffe, wie Benzol, Toluol, Xylole, Petroläther oder Hexan; halogenierte Kohlenwasserstoffe, wie Chlorbenzol, Dichlormethan, Aethylenchlorid, Trichlormethan, Tetrachlormethan oder Tetrachloräthylen; Aether und ätherartige Verbindungen, wie Dialkyläther (Diäthyläther, Diisopropyläther, tert.-Butylmethyläther, etc.), Anisol, Dioxan oder Tetrahydrofuran; Nitrile, wie Acetonitril oder Propionitril; Ester, wie Aethylacetat, Propylacetat oder Butylacetat; Ketone, wie Aceton, Diäthylketon oder Methyläthylketon; und Gemische solcher Lösungsmittel untereinander. Man kann die Reaktion aber auch in einem Ueberschuss einer der oben genannten Basen durchführen oder anstelle der Base auch ein zweites Aequivalent oder auch einen grösseren Ueberschuss der Verbindung III einsetzen.

Geeignete Kondensationsmittel für die <u>Verfahrensvariante b)</u> sind z. B. Phosphorigsäure-dichlorid-phenylester, Benzolphosphonsäure-dichlorid, 2,4,6-Trichlor- 1,3,5-triazin, 5,6-Dioxo- 1,3-diphenyl-5,6-dihydro-<thieno[3,4-b]- 1,4-dioxin>-2,2-dioxid, Kohlensäurediimidazolid, Dicyclohexylcarbodiimid, Aluminiumoxid, Titan(IV)chlorid, 2,2,4,4,6,6-Hexachlor-1,3,5,2,4,6-triazatriphosphorin und Chlorameisensäureniederalkylester, wie Chlorameisensäureisobutylester. Man arbeitet bevorzugt in Gegenwart einer Base, beispielsweise in Gegenwart eines organischen Amins, z. B. eines Trialkylamins (wie Trimethylamin, Triäthylamin, Tripropylamin oder Diisopropyläthylamin), eines Pyridins (wie Pyridin, 4-Dimethylaminopyridin oder 4-Pyrrolidinopyridin), eines Morpholins (wie N-Methylmorpholin) oder eines N,N-Dialkylanilins (wie N,N-Dimethylanilin oder N-Methyl-N-äthylanilin). Man arbeitet zweckmässigerweise in Gegenwart eines inerten Lösungsmittels oder Lösungsmittelgemisches und bei Temperaturen von -30 bis +70°C, vorzugsweise von - 10 bis +50°C. Als Lösungsmittel eignen sich beispielsweise aliphatische und aromatische Kohlenwasserstoffe, wie Benzol, Toluol, Xylole, Petroläther oder Hexan; halogenierte Kohlenwasserstoffe, wie Chlorbenzol, Dichlormethan, Aethylenchlorid, Trichlormethan, Tetrachlormethan oder Tetrachloräthylen; Aether und ätherartige Verbindungen, wie Dialkyläther (Diäthyläther, Diisopropyläther, tert.-Butylmethyläther etc.), Anisol, Dioxan oder Tetrahydrofuran; Nitrile, wie Acetonitril oder Propionitril; Ester, wie Aethylacetat, Propylacetat oder Butylacetat; und Gemische solcher Lösungsmittel untereinander.

Bei der <u>Verfahrensvariante c)</u> werden die Reaktanden mit Vorteil in einem inerten Lösungsmittel oder Lösungsmittelgemisch umgesetzt. Es eignen sich hierfür beispielsweise aliphatische und aromatische Kohlenwasserstoffe, wie Benzol, Toluol, Xylole, Petroläther oder Hexan; halogenierte Kohlenwasserstoffe, wie Chlorbenzol, Dichlormethan, Aethylenchlorid, Trichlormethan, Tetrachlormethan oder Tetrachloräthylen; Aether und ätherartige Verbindungen, wie Dialkyläther (Diäthyläther, Diisopropyläther, tert.-Butylmethyläther etc.), Anisol,

Dioxan oder Tetrahydrofuran; Nitrile, wie Acetonitril oder Propionitril; Alkohole, wie Methanol, Aethanol, Propanol oder Isopropanol; und Wasser. Die Aminkomponente III wird mit Vorteil im Ueberschuss verwendet. Die Reaktionstemperaturen liegen in der Regel zwischen 0 und +120°C.

Die Umwandlung von freien Verbindungen I in Salze und von Salzen in freie Verbindungen I oder in andere Salze erfolgt in üblicher Weise, z. B. durch Behandlung einer freien Verbindung I mit einer Säure oder eines Salzes mit einer Base.

Die Verbindungen II, III, IV und V sind bekannt oder können in Analogie zu den bekannten Verbindungen hergestellt werden.

Die erfindungsgemässen Verbindungen I sind bei günstiger Warmblüter-, Fisch- und Pflanzenverträglichkeit wertvolle Wirkstoffe auf dem Gebiet der Schädlingsbekämpfung. Insbesondere wirken die erfindungsgemässen Wirkstoffe gegen Insekten und Spinnentiere, wie sie an Nutz- und Zierpflanzen in der Landwirtschaft und im Gartenbau, insbesondere in Reis-, Baumwoll-, Gemüse- und Obstpflanzungen, und im Forst vorkommen. Die Verbindungen I eignen sich besonders zur Bekämpfung von Insekten in Reis-, Obst- und Gemüsekulturen, insbesondere von pflanzenschädigenden Insekten, wie Aphis craccivora, Nilaparvata lugens und Nephotettix cincticeps. Weitere Anwendungsgebiete der erfindungsgemässen Wirkstoffe sind der Vorrats- und Materialschutz sowie im Hygienesektor insbesondere der Schutz von Haus- und Nutztieren. Die Verbindungen I sind gegen alle oder einzelne Entwicklungsstadien von normal sensiblen, aber auch von resistenten Arten von Schädlingen wirksam. Dabei kann sich ihre Wirkung z. B. in einer Abtötung der Schädlinge, welche unmittelbar oder erst nach einiger Zeit, beispielsweise bei einer Häutung, eintritt, oder in einer verminderten Eiablage und/oder Schlupfrate zeigen.

Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prags spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung Orthoptera zum Beispiel
Blatta spp., Blattella spp., Gryllotalpa spp., Leucophaea maderae, Locusta spp., Periplaneta spp. und Schistocerca spp.;
aus der Ordnung Isoptera zum Beispiel
Reticulitermes spp.;
aus der Ordnung Psocoptera zum Beispiel
Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum,

Empoasca spp., Eriosoma larigerum, Erythroneura spp., Gascardia spp., Laodelphax spp., Lecanium comi, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis;
aus der Ordnung Thysanura zum Beispiel
Lepisma saccharina und
aus der Ordnung Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp..

Die gute pestizide Wirkung der erfindungsgemässen Verbindungen I entspricht einer Abtötungsrate (Mortalität) von mindestens 50-60 % der erwähnten Schädlinge.

Die Wirkung der erfindungsgemässen Verbindungen I und der sie enthaltenden Mittel lässt sich durch Zusatz von anderen Insektiziden und/oder Akariziden wesentlich verbreitem und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln und Granulaten, auch zu Verkapselungen in polymeren Stoffen, in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden als auch der Wirkstoff beim Säen in die Saatfurche appliziert werden.

Die Formulierungen, das heisst die den Wirkstoff der Formel I, beziehungsweise eine Kombination dieses Wirkstoffs mit anderen Insektiziden und/oder Akariziden, und gegebenenfalls feste oder flüssige Hilfsstoffe enthaltenden Mittel, Zubereitungen oder Zusammensetzungen, werden in bekannter Weise hergestellt, zum Beispiel durch inniges Vermischen und/oder Vermahlen des Wirkstoffs mit den Hilfsstoffen, wie Streckmitteln, z. B. Lösungsmitteln oder festen Trägerstoffen, oder wie oberflächenaktiven Verbindungen (Tensiden).

Als Lösungsmittel können in Frage kommen: Aromatische Kohlenwasserstoffe, bevorzugt die Fraktionen $C_8$ bis $C_{12}$ von Alkylbenzolen, wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetanolalkohol, stark polare Lösungsmittel, wie N-Methyl-2-pyrrolidon Dimethylsulfoxid oder N,N-Dimethylformamid, Wasser sowie gegebenenfalls epoxidierte Pflanzenöle, wie gegebenenfalls epoxidiertes Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmotillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden.

Als oberflächenaktive Verbindungen kommen je nach Art des zu formulierenden Wirkstoffs I oder der Kombination dieses Wirkstoffs mit anderen Insektiziden und/oder Akariziden nichtionische, kationische und/oder anionische Tenside mit guten Emulgier-, Dispergier- und Netzeigenschaften in Betracht. Unter Tensiden sind auch Tensidgemische zu verstehen.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten. Als Beispiele nichtionischer Tenside seien Nonylphenolpoly- äthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat, in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, Beispiele sind das Stearyltrimethylammoniumchlorid oder das Benzyl-di-(2-chloräthyl)-äthylammoniumbromid.

Geeignete anionische Tenside können sowohl sogenannte wasserlösliche Seifen als auch wasserlösliche synthetische oberflächenaktive Verbindungen sein. Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure oder von natürlichen Fettsäuregemischen, die beispielsweise aus Kokosnuss- oder Tallöl gewonnen werden können; ferner sind auch die Fettsäuremethyl-taurin-salze zu erwähnen. Häufiger werden jedoch sogenannte synthetische Tenside verwendet, insbesondere Fettsulfonate, Fettsulfate, sulfonierte Benzimidazolderivate oder Alkylarylsulfonate. Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolammoniumsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie Salze des Phosphorsäureesters eines p-Nonylphenol-(4- 14)-Aethylenoxid-Adduktes oder Phospholipide, in Frage.

Die vorstehend aufgeführten Tenside sind nur als Beispiele anzusehen; in der einschlägigen Literatur werden viele weitere in der Formulierungstechnik gebräuchliche und erfindungsgemäss geeignete Tenside beschrieben.

Die pestiziden Zubereitungen enthalten in der Regel 0,1 bis 99% , insbesondere 0,1 bis 95% , an Wirkstoff I oder an der Kombination dieses Wirkstoffs mit anderen Insektiziden und/oder Akariziden und 1 bis 99,9% , insbesondere 5 bis 99,9% , eines festen oder flüssigen Hilfsstoffes, wobei in der Regel 0 bis 25% , insbesondere 0,1 bis 20% , der Zubereitungen Tenside sein können (% bedeutet jeweils Gewichtsprozent). Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent):

Emulgierbare Konzentrate:

| | |
|---|---|
| Wirkstoff: | 1 bis 90 % , bevorzugt 5 bis 20 % |
| Tensid: | 1 bis 30 % , vorzugsweise 10 bis 20 % |

flüssiger Trägerstoff: 5 bis 94 % , vorzugsweise 70 bis 85 %

Stäube:

Wirkstoff: 0,1 bis 10 % , vorzugsweise 0,1 bis 1 %
fester Trägerstoff: 99,9 bis 90 % , vorzugsweise 99,9 bis 99 %

Suspensions-Konzentrate:

Wirkstoff: 5 bis 75 % , vorzugsweise 10 bis 50 %
Wasser: 94 bis 24 % , vorzugsweise 88 bis 30 %
Tensid: 1 bis 40 % , vorzugsweise 2 bis 30 %

Benetzbare Pulver:

Wirkstoff: 0,5 bis 90 % , vorzugsweise 1 bis 80 %
Tensid: 0,5 bis 20 % , vorzugsweise 1 bis 15 %
fester Trägerstoff: 5 bis 95 % , vorzugsweise 15 bis 90 %

Granulate:

Wirkstoff: 0,5 bis 30 % , vorzugsweise 3 bis 15 %
fester Trägerstoff: 99,5 bis 70 % , vorzugsweise 97 bis 85 %

Die Zubereitungen können auch weitere Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Dünger oder andere Wirkstoffe zur Erzielung spezieller Effekte enthalten.

Die folgenden Beispiele dienen der Erläuterung der Erfindung. Sie schränken die Erfindung nicht ein. Temperaturen sind in Grad Celsius angegeben.

Herstellungsbeispiele

Beispiel H1:

4-Chlor-4,4-difluor-N-methoxy-buttersäureamid (Tabelle 1, Verbindung Nr. 1.56).

$$ClF_2C\text{-}CH_2\text{-}CH_2\text{-}CO\text{-}NH\text{-}OCH_3$$

Zu einer Lösung von 5.0 g 4-Chlor-4,4-difluor-buttersäure in 50 ml Tetrahydrofuran werden bei 0°C zunächst 2.89 g N-Methylmorpholin und anschliessend 4.53 g Chlorameisensäureisobutylester tropfenweise zugegeben. Anschliessend lässt man das Reaktionsgemisch 1 Stunde bei 0°C rühren und versetzt es dann mit 2.63 g O-Methyl-hydroxylamin-hydrochlorid und 3.51 g N-Methylmorpholin. Nach 16-stündigem Rühren bei Raumtemperatur wird das Lösungsmittel im Vakuum abgedampft und der erhaltene Rückstand in einem Gemisch aus Essigsäureäthylester und 1 N Salzsäure aufgenommen. Die organische Phase wird abgetrennt, nacheinander mit gesättigter $NaHCO_3$- und gesättigter NaCl-Lösung gewaschen, über $MgSO_4$ getrocknet und eingedampft. Das erhaltene Rohprodukt lässt sich durch Kugelrohrdestillation (90°C, 4 mbar) reinigen. Man erhält so das 4-Chlor-4,4-difluor-N-methoxy-buttersäureamid in Form eines farblosen Oels (Brechungsindex $n_D^{22}$ = 1.4283).

Beispiel H2:

4-Chlor-4,4-difluor-N-(phenylaminocarbonylamino)-buttersäureamid (Tabelle 1, Verbindung Nr. 1.50).

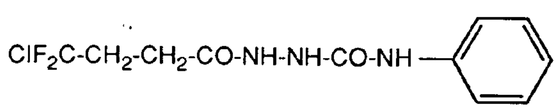

Zu einem Gemisch aus 3.42 g 4-Phenylsemicarbazid, 4.47 g Pyridin und 50 ml Toluol lässt man bei 0°C 4.0 g 4-Chlor-4,4-difluor-buttersäurechlorid innerhalb von 1 Stunde zutropfen. Nach 16-stündigem Rühren bei

Raumtemperatur wird die erhaltene Suspension filtriert. Der Kristallbrei wird mit Wasser und Diäthyläther gewaschen. Man erhält so das 4-Chlor-4,4-difluor-N-(phenylaminocarbonylamino)-buttersäureamid in Form eines farblosen Kristallisats (Smp.: 180- 181°C).

Beispiel H3:

4-Chlor-4,4-difluor-N-(äthoxycarbonylamino)-buttersäureamid (Tabelle 1, Verbindung Nr. 1.47).

$$ClF_2C-CH_2-CH_2-CO-NH-NH-CO-OC_2H_5$$

Zu einer Lösung von 2.35 g Hydrazincarbonsäureäthylester und 5,72 g Triäthylamin in 50 ml Toluol lässt man bei 0°C innerhalb von einer Stunde 4.0 g 4-Chlor-4,4-difluorbuttersäurechlorid zutropfen. Nach 16-stündigem Rühren bei Raumtemperatur wird das Reaktionsgemisch mit einem Gemisch aus Essigsäureäthylester und 1 N Salzsäure versetzt. Die organische Phase wird abgetrennt, nacheinander mit gesättigter $NaHCO_3$- und gesättigter NaCl-Lösung gewaschen, über $MgSO_4$ getrocknet und eingedampft. Das erhaltene Rohprodukt wird aus Toluol/Hexan umkristallisiert. Man erhält so das 4-Chlor-4,4-difluor-N-(äthoxycarbonylamino)-buttersäureamid (Smp.: 110-111°C).

Beispiel H4:

4-Chlor-4,4-difluor-N-(phenylcarbonylamino)-buttersäureamid (Tabelle 1, Verbindung Nr. 1.32).

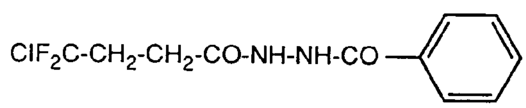

Zu einer Lösung von 3.0 g 4-Chlor-4,4-difluor-buttersäure und 1.72 g N-Methylmorpholin in 30 ml Tetrahydrofuran werden bei 0°C 2.72 g Chlorameisensäureisobutylester zugegeben. Anschliessend lässt man das Reaktionsgemisch 1 Stunde bei 0°C rühren und versetzt es dann innerhalb von 1 Stunde mit einer Lösung von 2.58 g Benzoylhydrazin und 0.19 g N-Methylmorpholin in 10 ml Tetrahydrofuran. Nach 16-stündigem Rühren bei Raumtemperatur wird das Lösungsmittel im Vakuum abgedampft und der erhaltene Rückstand in einem Gemisch aus Essigsäureäthylester und 1 N Salzsäure aufgenommen. Die organische Phase wird abgetrennt, nacheinander mit gesättigter $NaHCO_3$- und gesättigter NaCl-Lösung gewaschen, über $MgSO_4$ getrocknet und eingedampft. Das erhaltene Rohprodukt wird aus Toluol/Hexan umkristallisiert. Man erhält so das 4-Chlor-4,4-difluor-N-(phenylcarbonylamino)-buttersäureamid (Smp.: 153- 154°C).

Beispiel H5:

In analoger Weise wie in den Beispielen H1 bis H4 beschrieben können auch die anderen in der folgenden Tabelle 1 aufgeführten Verbindungen der Formel I hergestellt werden. In der Spalte "Physik. Daten" dieser Tabelle bezeichnen die angegebenen Temperaturen jeweils den Schmelzpunkt der betreffenden Verbindung und "$n_D^T$" steht für den Brechungsindex der betreffenden Verbindung bei der Temperatur T°C.

Tabelle 1

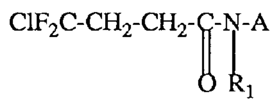

| Verb. Nr. | $R_1$ | A | Physik. Daten |
|---|---|---|---|
| 1.01 | H | $NH_2$ | |
| 1.02 | H | $N(CH_3)_2$ | 82-84°C |
| 1.03 | H | $N(CH_3)C_6H_5$ | 81-82°C |
| 1.04 | H | $NH-CH_3$ | |
| 1.05 | $CH_3$ | $NH_2$ | |
| 1.06 | H | $NH-CH_2CF_3$ | |
| 1.07 | $CH_2CF_3$ | $NH_2$ | |
| 1.08 | H | $NH-CH_2-C_6H_5$ | $n_D^{22} = 1.5100$ |
| 1.09 | $CH_2-C_6H_5$ | $NH_2$ | |
| 1.10 | H | $NH-C(CH_3)_3$ | |
| 1.11 | H | $NH-C_6H_5$ | 126-127°C |
| 1.12 | H | $NH-C_6H_4-CH_3-(2)$ | |
| 1.13 | H | $NH-C_6H_4-CH_3-(4)$ | |
| 1.14 | H | 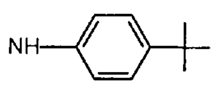 | |
| 1.15 | H | 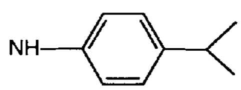 | |
| 1.16 | H | $NH-C_6H_4-Cl-(2)$ | |
| 1.17 | H | $NH-C_6H_4-Cl-(4)$ | |
| 1.18 | H | $NH-C_6H_4-F-(4)$ | |
| 1.19 | H | $NH-C_6H_4-OCH_3-(4)$ | 96-97°C |
| 1.20 | H | $NH-C_6H_4-Br-(4)$ | |
| 1.21 | H | 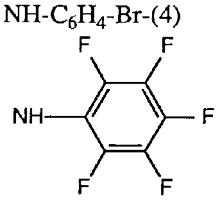 | |

Tabelle 1 (Fortsetzung):

| Verb. Nr. | $R_1$ | A | Physik. Daten |
|---|---|---|---|
| 1.22 | H | 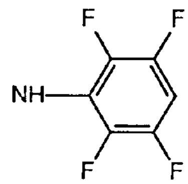 | 110.5-111.5°C |
| 1.23 | H | 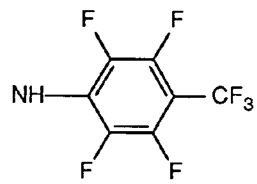 | |
| 1.24 | H | $NH-C_6H_4-NO_2-(2)$ | |
| 1.25 | H | $NH-C_6H_4-NO_2-(3)$ | |
| 1.26 | H | $NH-C_6H_4-NO_2-(4)$ | 139-140°C |
| 1.27 | H | 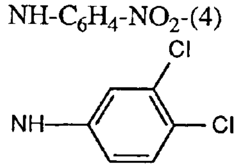 | |
| 1.28 | H | $N(C_6H_5)_2$ | 152-154°C |
| 1.29 | H | $NH-CO-CH_3$ | 166-168°C |
| 1.30 | H | $NH-CO-CH_2-C_6H_5$ | |
| 1.31 | H | $NH-CO-CH_2CN$ | |
| 1.32 | H | $NH-CO-C_6H_5$ | 153-154°C |
| 1.33 | H | $NH-CO-C_6H_4-NO_2-(2)$ | |
| 1.34 | H | $NH-CO-C_6H_4-NO_2-(4)$ | 193-194°C |
| 1.35 | H | $NH-CO-C_6H_4-OCH_3-(3)$ | |
| 1.36 | H | $NH-CO-C_6H_4-Br-(3)$ | |
| 1.37 | H | $NH-CO-C_6H_4-Cl-(2)$ | |
| 1.38 | H | $NH-CO-C_6H_4-Cl-(4)$ | |
| 1.39 | H | $NH-CO-C_6H_4-CH_3-(4)$ | |
| 1.40 | H | 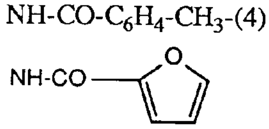 | 142°C |

Tabelle 1 (Fortsetzung):

| Verb. Nr. | $R_1$ | A | Physik. Daten |
|---|---|---|---|
| 1.41 | H | 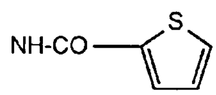 | 178-179°C |
| 1.42 | H | 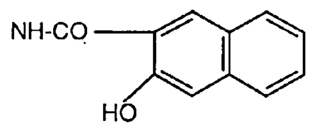 | |
| 1.43 | H | 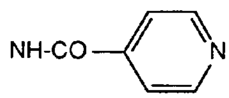 | |
| 1.44 | H | 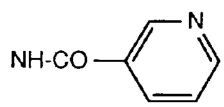 | 168-178°C |
| 1.45 | H | $N[C(CH_3)_3]CO-C_6H_5$ | 137-138°C |
| 1.46 | H | $NH-COO-CH_3$ | |
| 1.47 | H | $NH-COO-CH_2CH_3$ | 110-111°C |
| 1.48 | H | $NH-COO-C(CH_3)_3$ | 76-77°C |
| 1.49 | H | 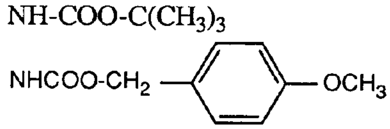 | 88-89°C |
| 1.50 | H | $NHCONH-C_6H_5$ | 180-181°C |
| 1.51 | H | $NHCON(C_6H_5)_2$ | |
| 1.52 | H | $NHCONH_2$ | |
| 1.53 | H | 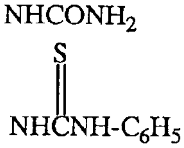 | 150-151°C |
| 1.54 | H | 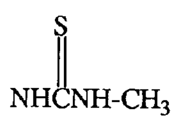 | 162-163°C |
| 1.55 | H | 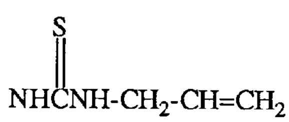 | |
| 1.56 | H | $O-CH_3$ | $n_D^{22} = 1.4283$ |

Tabelle 1 (Fortsetzung):

| Verb. Nr. | $R_1$ | A | Physik. Daten |
|---|---|---|---|
| 1.57 | H | $O-CH_2-C_6H_5$ | |
| 1.58 | H | $O-CH_2-C_6H_4-NO_2-(4)$ | 106-110°C |
| 1.59 | H | 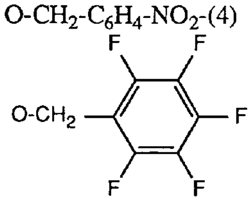 | 48-51°C |
| 1.60 | H | $O-CH_2CH_3$ | |
| 1.61 | H | $O-C(CH_3)_3$ | |
| 1.62 | H | $O-C_6H_5$ | |
| 1.63 | H | $O-CH_2CH=CH_2$ | |
| 1.64 | H | 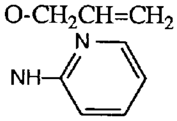 | |
| 1.65 | H | 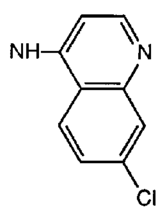 | |
| 1.66 | H | 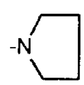 | |
| 1.67 | H | 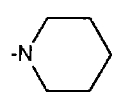 | 134-135°C |
| 1.68 | H | 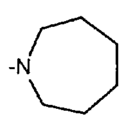 | |
| 1.69 | H | 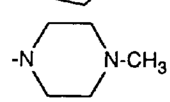 | 163-164°C |

Tabelle 1 (Fortsetzung):

| Verb. Nr. | $R_1$ | A | Physik. Daten |
|---|---|---|---|
| 1.70 | H | 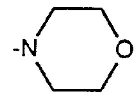 | |
| 1.71 | H | 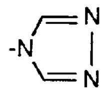 | |
| 1.72 | H | 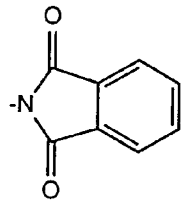 | 142-144°C |
| 1.73 | H | $N(C_6H_5)COCH_2CH_2CClF_2$ | Harz |

Formulierungsbeispiele (% = Gewichtsprozent)

| Beispiel F1: Emulsions-Konzentrate | a) | b) | c) |
|---|---|---|---|
| Wirkstoff Nr. 1.56 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.56 | 80 % | 10 % | 5 % | 95 % |

| | | | | |
|---|---|---|---|---|
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.56 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Dichlormethan gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.56 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr. 1.47 | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyäthylenglykol-äther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

Beispiel F6: Emulsions-Konzentrat

| | |
|---|---|
| Wirkstoff Nr. 1.47 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 1.03 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Beispiel F8: Extruder-Granulat

| | |
|---|---|
| Wirkstoff Nr. 1.02 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

Beispiel F9: Umhüllungs-Granulat

| | |
|---|---|
| Wirkstoff Nr. 1.11 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

Beispiel F10: Suspensions-Konzentrat

| | |
|---|---|
| Wirkstoff Nr. 1.40 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Wirkung gegen Boophilus microplus

Vollgesogene adulte Zeckenweibchen werden auf eine PVC-Platte aufgeklebt und mit einem Wattebausch überdeckt. Zur Behandlung werden die Testtiere mit 10 ml einer wässrigen Testlösung, die 125 ppm Wirkstoff enthält, übergossen. Anschliessend wird der Wattebausch entfernt und die Zecken werden 4 Wochen zur Eiablage inkubiert. Die Wirkung gegen Boophilus microplus zeigt sich entweder beim Weibchen als Mortalität oder Sterilität oder bei den Eiern als ovizide Wirkung.
Verbindungen der Tabelle 1 zeigen in diesem Test gute Wirkung. Insbesondere zeigen die Verbindungen Nr. 1.02, 1.03, 1.11, 1.22, 1.29, 1.32, 1.40, 1.41, 1.44, 1.45, 1.47, 1.48, 1.49, 1.50, 1.54, 1.56, 1.67 und 1.69 eine Wirkung von mehr als 80%.

Beispiel B2: Wirkung gegen Crocidolmia binotalis

Junge Kohlpflanzen werden mit einer wässrigen Emulsions-Spritzbrühe, die 400 ppm Wirkstoff enthält, besprüht. Nach dem Antrocknen des Spritzbelages werden die Kohlpflanzen mit 10 Raupen des dritten Stadiums von Crocidolmia binotalis besiedelt und in einen Plastikbehälter gegeben. 3 Tage später erfolgt die Auswertung. Aus den Vergleichen der Anzahl toter Raupen und des Frassschadens zwischen den behandelten und den unbehandelten Pflanzen werden die prozentuale Reduktion der Population und die prozentuale Reduktion des Frassschadens (% Wirkung) bestimmt. Verbindungen der Tabelle 1 zeigen gute Wirkung in diesem Test. Insbesondere zeigt die Verbindung Nr. 1.32 eine Wirkung von mehr als 80%.

Beispiel B3: Wirkung gegen Aonidiella aurantii

Kartoffelknollen werden mit Wanderlarven ("Crawlern") von Aonidiella aurantii besiedelt. Nach etwa 2 Wochen werden die Kartoffeln in eine wässrige Emulsions-bzw. Suspensions-Spritzbrühe getaucht, die den Wirkstoff in einer Konzentration von 400 ppm enthält. Nach dem Abtrocknen der so behandelten Kartoffelknollen werden diese in einem Plastikbehälter inkubiert. Zur Auswertung wird 10 bis 12 Wochen später die Ueberlebensrate der Wanderlarven der ersten Folgegeneration der behandelten Population mit derjenigen von unbehandelten Kontrollansätzen verglichen. Verbindungen der Tabelle 1 zeigen in diesem Test gute Wirkung. Insbesondere zeigen die Verbindungen Nr. 1.03, 1.22, 1.47 und 1.56 eine Wirkung von mehr als 80%.

Beispiel B4: Wirkung gegen Nilaparvata lugens

Reispflanzen werden mit einer wässrigen Emulsions-Spritzbrühe, die 400 ppm Wirkstoff enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten und den unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.02, 1.03, 1.08, 1.11, 1.22, 1.26, 1.29, 1.32, 1.40, 1.41, 1.44, 1.45, 1.47, 1.48, 1.49, 1.50, 1.53, 1.54, 1.56, 1.58, 1.59, 1.67, 1.69 und 1.72 eine Wirkung von mehr als 80%.

Beispiel B5: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm Wirkstoff enthält, besprüht. Die Pflanzen werden anschliessend 6 Tage bei 25°C inkubiert und danach ausgewertet. Aus den Vergleichen der Anzahl toter Eier, Larven und Adulten auf den behandelten und den unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.48, 1.49 und 1.67 eine Wirkung von mehr als 80%.

Beispiel B6: Wirkung gegen Anthonomus grandis

Junge Baumwollpflanzen werden mit einer wässrigen Emulsions-Spritzbrühe, die 400 ppm Wirkstoff enthält, besprüht. Nach dem Antrocknen des Spritzbelages werden die Baumwollpflanzen mit 10 Adulten von Anthonomus grandis besiedelt und in einen Plastikbehälter gegeben. 3 Tage später erfolgt die Auswertung. Aus den Vergleichen der Anzahl toter Käfer und des Frassschadens zwischen den behandelten und den unbehandelten Pflanzen werden die prozentuale Reduktion der Population und die prozentuale Reduktion des Frassschadens (% Wirkung) bestimmt.
Verbindungen der Tabelle 1 zeigen gute Wirkung in diesem Test. Insbesondere zeigen die Verbindungen Nr. 1.02, 1.11, 1.29, 1.32, 1.47, 1.49 und 1.67 eine Wirkung von mehr als 80%.

Beispiel B7: Wirkung gegen Aphis craccivora

Erbsenkeimlinge werden mit Aplis craccivora infiziert, anschliessend mit einer Spritzbrühe, die 400 ppm Wirkstoff enthält, besprüht und dann bei 20°C inkubiert. 3 und 6 Tage später erfolgt die Auswertung. Aus dem Vergleich der Anzahl toter Blattläuse auf den behandelten und den unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.02, 1.03, 1.11, 1.29, 1.32, 1.47, 1.48, 1.50, 1.53, 1.56 und 1.67 eine Wirkung von mehr als 80%.

Beispiel B8: Systemische Wirkung gegen Myzus persicae

Erbsenkeimlinge werden mit Myzus persicae infiziert, anschliessend mit den Wurzeln in eine Spritzbrühe, die 400 ppm Wirkstoff enthält, gestellt und dann bei 20°C inkubiert. 3 und 6 Tage später erfolgt die Auswertung. Aus dem Vergleich der Anzahl toter Blattläuse auf den behandelten und den unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.02, 1.03, 1.11, 1.22, 1.29, 1.32, 1.47, 1.48, 1.50 und 1.56 eine Wirkung von mehr als 80%.

Beispiel B9: Systemische Wirkung gegen Nilaparvata lugens

Töpfe mit Reispflanzen werden in eine wässrige Emulsionslösung, die 400 ppm Wirkstoff enthält, gestellt. Anschliessend werden die Pflanzen mit Larven des 2. und 3. Stadiums besiedelt. 6 Tage später erfolgt die Auswertung. Aus dem Vergleich der Anzahl Zikaden auf den behandelten und den unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.02, 1.03, 1.08, 1.11, 1.19, 1.22, 1.26, 1.29, 1.32, 1.34, 1.40, 1.41, 1.44, 1.47, 1.48, 1.49, 1.50, 1.56, 1.58, 1.59, 1.67, 1.69 und 1.72 eine Wirkung von mehr als 80%.

Beispiel B 10: Ovo/larvizide Wirkung auf Heliothis virescens

Auf Baumwolle abgelegte Eier von Heliothis virescens werden mit einer wässrigen Emulsions-Spritzbrühe, die 400 ppm Wirkstoff enthält, besprüht. Nach 8 Tagen werden der prozentuale Schlupf der Eier und die Ueberlebensraten der Raupen im Vergleich zu unbehandelten Kontrollansätzen ausgewertet (% Reduktion der Population).
Verbindungen der Tabelle 1 zeigen in diesem Test gute Wirkung. Insbesondere zeigen die Verbindungen Nr. 1.03, 1.08, 1.11, 1.59 und 1.67 eine Wirkung von mehr als 80%.

Beispiel B11: Systemische Wirkung gegen Nephotettix cincticeps

Töpfe mit Reispflanzen werden in eine wässrige Emulsionslösung, die 400 ppm Wirkstoff enthält, gestellt. Anschliessend werden die Pflanzen mit Larven des 2. und 3. Stadiums besiedelt. 6 Tage später erfolgt die Auswertung. Aus dem Vergleich der Anzahl Zikaden auf den behandelten und den unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.03, 1.29, 1.41, 1.48 und 1.49 eine Wirkung von mehr als 80%.

Beispiel B12: Wirkung gegen Heliothis virescens

Junge Sojapflanzen werden mit einer wässrigen Emulsions-Spritzbrühe, die 400 ppm Wirkstoff enthält, besprüht. Nach dem Antrocknen des Spritzbelages werden die Pflanzen mit 10 Raupen des ersten Stadiums von Heliothis virescens besiedelt und in einen Plastikbehälter gegeben. 6 Tage später erfolgt die Auswertung. Aus den Vergleichen der Anzahl toter Raupen und des Frassschadens zwischen den behandelten und den unbehandelten Pflanzen werden die prozentuale Reduktion der Popularion und des Frasssschadens (% Wirkung) bestimmt.
Verbindungen der Tabelle 1 zeigen gute Wirkung in diesem Test. Insbesondere zeigen die Verbindungen Nr. 1.11, 1.32, 1.47 und 1.56 eine Wirkung von mehr als 80%.

Beispiel B13: Wirkung gegen Nephotettix cincticeps

Reispflanzen werden mit einer wässrigen Emulsions-Spritzbrühe, die 400 ppm Wirkstoff enthält, behandelt. Nach dem Antrocknen des Spritzbelages werden die Pflanzen mit Zikadenlarven des 2. und 3. Stadiums besiedelt. 21 Tage später erfolgt die Auswertung. Aus dem Vergleich der Anzahl überlebender Zikaden auf den behandelten und den unbehandelten Pflanzen wird die prozentuale Reduktion der Population (% Wirkung) bestimmt.
Verbindungen der Tabelle 1 zeigen gute Wirkung in diesem Test. Insbesondere zeigen die Verbindungen Nr. 1.29, 1.40, 1.56 und 1.67 eine Wirkung von mehr als 80%.

Beispiel B14: Wirkung gegen Ctenocephalides felis

20 bis 25 Floheier werden in eine waagerecht stehende 50 ml-Zellkulturflasche gegeben, in der zuvor 15 g Flohlarven-Nährmedium, welches 100 ppm Wirkstoff enthält, vorgelegt wurden. Die Flaschen werden in einem Brutschank bei 26-27°C und 60-70 % Luftfeuchtigkeit bebrütet. Nach 21 Tagen wird die Anwesenheit von adulten Flöhen, nicht geschlüpften Puppen und Larven überprüft.
Verbindungen der Tabelle 1 zeigen gute Wirkung in diesem Test.

## Patentansprüche

1. Eine Verbindung der Formel

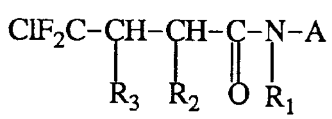

(I),

worin

A einen unsubstituierten oder substituierten, über ein Ringstickstoffatom gebundenen, aromatischen oder nicht-aromarischen, mono- oder bicyclischen, stickstoffhaltigen Heterocyclus oder eine Gruppe $-OR_4$, $-N(R_4)R_5$, $-N(R_5)COR_4$, $-N(R_5)COOR_4$, $-N(R_5)CON(R_4)R_6$ oder $-N(R_5)CSN(R_4)R_6$;
$R_1$ Wasserstoff, $C_1$-$C_6$-Alkyl, $C_1$-$C_6$-Halogenalkyl oder Benzyl;
$R_2$ und $R_3$ unabhängig voneinander Wasserstoff oder $C_1$-$C_4$-Alkyl;
$R_4$ Wasserstoff, eine unsubstituierte oder substituierte $C_1$-$C_{20}$-Alkyl-, $C_3$-$C_{10}$-Alkenyl-, $C_3$-$C_{10}$-Alkinyl-, $C_3$-$C_7$-Cycloalkyl- oder Aryl-Gruppe oder einen unsubstituierten oder substituierten, aromatischen oder nicht-aromatischen, über ein Ringkohlenstoffatom gebundenen, mono- oder bicyclischen, Heterocyclus; und
$R_5$ und $R_6$ unabhängig voneinander Wasserstoff, $C_1$-$C_6$-Alkyl oder Phenyl bedeuten, in freier Form oder in Salzform.

2. Eine Verbindung gemäss Anspruch 1 der Formel I in freier Form.

3. Eine Verbindung gemäss Anspruch 2 der Formel I, worin A für $-OR_4$, $-N(H)R_4$, $-N(CH_3)R_4$, $-N(C_6H_5)R_4$, $-N(H)COR_4$, $-N(CH_3)COR_4$, $-N(H)COOR_4$, $-N(H)CON(H)R_4$, $-N(H)CON(C_6H_5)R_4$, $-N(H)CSN(H)R_4$, Pyrrolidin-1-yl, Piperidin-1-yl, Perhydroazepin-1-yl, Morpholin-4-yl, 4-Methylpiperazin-1-yl, Triazol-N-yl oder Phthalimid-N-yl steht.

4. Eine Verbindung gemäss Anspruch 2 der Formel I, worin $R_4$ für $C_1$-$C_{10}$-Alkyl; $C_3$-$C_7$-Cycloalkyl; $C_3$-$C_{10}$-Alkenyl; $C_3$-$C_{10}$-Alkinyl; Aryl; $C_3$-$C_{10}$-Halogenalkenyl; $C_3$-$C_{10}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyano, Benzoyl, Halogenbenzoyl, Phenoxy, Halogenphenoxy, $C_1$-$C_4$-Alkylphenoxy, $C_1$-$C_4$-Halogenalkylphenoxy, $C_1$-$C_4$-Alkylamino, Di-$C_1$-$C_4$-alkylamino; Anilino, N-$C_1$-$C_4$-Alkylanilino, N-$C_1$-$C_6$-Alkylcarbonylanilino, Phenylthio oder Halogenphenylthio substituiertes Aryl; einen über ein Ringkohlenstoffatom gebundenen, aromatischen oder nicht-aromatischen, mono- oder bicyclischen, Heterocyclus, welcher unsubstituiert oder durch Halogen, $C_1$-$C_4$-Alkyl, Nitro, $C_1$-$C_4$-Halogenalkyl, $C_1$-$C_4$-Halogenalkoxy, $C_1$-$C_4$-Alkoxy, $C_1$-$C_4$-Alkylthio oder Cyclopropyl substituiert sein kann; oder durch Hydroxy, Halogen, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Aryl, Aryloxy, Arylthio, Arylsulfonyl, Arylsufinyl, Arylsulfonyloxy, Arylcarbonyl oder Pyridyl substituiertes $C_1$-$C_{20}$-Alkyl, wobei die Aryl- und Pyridylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro, Cyano, Phenoxy, Halogenphenoxy, Phenylthio oder Halogenphenylthio substituiert sein können, steht.

5. Eine Verbindung gemäss Anspruch 2 der Formel I, worin $R_1$ Wasserstoff ist.

6. Eine Verbindung gemäss Anspruch 2 der Formel I, worin $R_2$ und $R_3$ Wasserstoff sind.

7. Eine Verbindung gemäss Anspruch 2 der Formel I, worin $R_4$ Wasserstoff; $C_1$ -$C_4$-Alkyl; durch Halogen oder $C_1$-$C_4$-Alkoxy substituiertes $C_1$-$C_4$-Alkyl; Phenyl; durch 1 bis 5 Halogenatome oder 1 bis 2 Substituenten, ausgewählt aus der Gruppe, bestehend aus $C_1$-$C_4$-Alkyl, $C_1$-$C_4$-Alkoxy, Cyano, $C_1$-$C_4$-Halogenalkyl und Nitro, substituiertes Phenyl; Benzyl; durch 1 bis 5 Halogenatome oder 1 bis 2 Substituenten, ausgewählt aus der Gruppe, bestehend aus $C_1$-$C_4$-Alkoxy und Nitro, substituiertes Benzyl; Thienyl; oder Furyl bedeutet.

8. Eine Verbindung gemäss Anspruch 2 der Formel I, worin $R_1$, $R_2$ und $R_3$ Wasserstoff bedeuten, A Piperidin-1-yl, 4-$C_1$-$C_4$-Alkylpiperazin-1-yl, Phthalimid-N-yl oder eine Gruppe $-OR_4$, $-N(R_4)R_5$, $-N(R_5)COR_4$, $-N(R_5)COOR_4$, $-N(R_5)CON(R_4)R_5$ oder $-N(R_5)CSN(R_4)R_6$ ist, $R_4$ $C_1$-$C_4$-Alkyl; Halogen-$C_1$-$C_4$-alkyl mit 1 bis und mit 3 Halogenatomen; Phenyl; durch 1 bis 5 Halogenatome oder 1 bis 2 Substituenten, ausgewählt aus der Gruppe, bestehend aus $C_1$-$C_4$-Alkoxy und Nitro, substituiertes Phenyl; Benzyl; durch 1 bis 5 Halogenatome oder 1 bis 2 Substituenten, ausgewählt aus der Gruppe, bestehend aus $C_1$-$C_4$-Alkoxy und Nitro, substituiertes Benzyl; Pyridyl; Thienyl; oder Furyl bedeutet, $R_5$ Wasserstoff, $C_1$-$C_4$-Alkyl oder Phenyl ist und $R_5$ Wasserstoff ist.

9. Eine Verbindung gemäss Anspruch 2 der Formel I, worin A $-OR_4$, $-N(R_4)R_5$, $-N(R_5)COR_4$ oder $-N(R_5)COOR_4$ ist und $R_5$ Wasserstoff, Methyl oder Phenyl ist.

**10.** Eine Verbindung gemäss Anspruch 8 der Formel I, worin $R_1$, $R_2$ und $R_3$ Wasserstoff bedeuten, A eine Gruppe $-N(R_4)R_5$, $-N(R_5)COR_4$ oder $-N(R_5)COOR_4$ ist, $R_4$ $C_1$-$C_4$-Alkyl, Halogen-$C_1$-$C_4$-alkyl mit 1 bis und mit 3 Halogenatomen, Phenyl, durch 1 bis 5 Halogenatome substituiertes Phenyl oder durch $C_1$-$C_4$-Alkoxy substituiertes Benzyl bedeutet und $R_5$ Wasserstoff oder Phenyl ist.

**11.** Eine Verbindung gemäss Anspruch 2 der Formel I, ausgewählt aus der Gruppe, bestehend aus den Verbindungen
(a) 4-Chlor-4,4-difluor-N-methoxy-buttersäureamid,
(b) 4-Chlor-4,4-difluor-N-(äthoxycarbonylamino)-buttersäureamid,
(c) 4-Chlor-4,4-difluor-N-(phenylamino)-buttersäureamid,
(d) 4-Chlor-4,4-difluor-N-(phenylcarbonylamino)-buttersäureamid,
(e) 4-Chlor-4,4-difluor-N-(phenylaminocarbonylamino)-buttersäureamid,
(f) 4-Chlor-4,4-difluor-N-(N,N-dimethylamino)-buttersäureamid,
(g) 4-Chlor-4,4-difluor-N-(methylcarbonylamino)-buttersäureamid,
(h) 4-Chlor-4,4-difluor-N-(fur-2-ylcarbonylamino)-buttersäureamid,
(i) 4-Chlor-4,4-difluor-N-(4-methoxybenzyloxycarbonylamino)-buttersäureamid,
(j) 4-Chlor-4,4-difluor-N-(thien-2-ylcarbonylamino)-buttersäureamid,
(k) 4-Chlor-4,4-difluor-N-(tert.-butoxycarbonylamino)-buttersäureamid und
(l) 4-Chlor-4,4-difluor-N-(N-methyl-N-phenyl-amino)-buttersäureamid.

**12.** Verfahren zur Herstellung einer Verbindung gemäss Anspruch 1 der Formel I, in freier Form oder in Salzform, dadurch gekennzeichnet, dass man
a) eine Verbindung der Formel

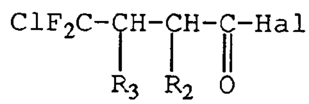
(II),

worin $R_2$ und $R_3$ die für die Formel I angegebenen Bedeutungen haben und Hal für Halogen, vorzugsweise Chlor oder Brom, steht, in Gegenwart einer Base mit einer Verbindung der Formel

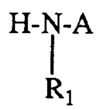
(III),

worin A und $R_1$ die für die Formel I angegebenen Bedeutungen haben, oder einem Salz davon umsetzt oder
b) eine Verbindung der Formel

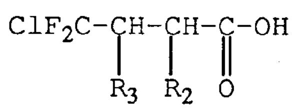
(IV),

worin $R_2$ und $R_3$ die für die Formel I angegebenen Bedeutungen haben, in Gegenwart eines Kondensationsmittels mit einer Verbindung der Formel III oder einem Salz davon umsetzt oder
c) eine Verbindung der Formel

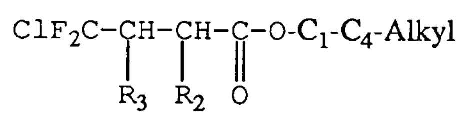
(V),

worin $R_2$ und $R_3$ die für die Formel I angegebenen Bedeutungen haben, mit einer Verbindung der Formel III oder einem Salz davon umsetzt und jeweils, wenn erwünscht, eine verfahrensgemäss erhältliche freie Verbindung der Formel I in ein Salz oder ein verfahrensgemäss erhältliches Salz einerVerbindung

der Formel I in die freie Verbindung der Formel I oder in ein anderes Salz überführt.

**13.** Schädlingsbekämpfungsmittel, dadurch gekennzeichnet, dass es mindestens eine Verbindung gemäss Anspruch 1 der Formel I, in freier Form oder in agrochemisch verwendbarer Salzform, als Wirkstoff und mindestens einen Hilfsstoff enthält.

**14.** Mittel gemäss Anspruch 13 zur Bekämpfung von Insekten und/oder Spinnentieren.

**15.** Verfahren zur Herstellung eines Mittels gemäss Anspruch 13 oder 14, dadurch gekennzeichnet, dass man den Wirkstoff mit dem (den) Hilfsstoff(en) innig vermischt.

**16.** Verwendung einer Verbindung gemäss Anspruch 1 der Formel I, in freier Form oder in agrochemisch verwendbarer Salzform, oder eines Mittels gemäss Anspruch 13 oder 14 zur Bekämpfung von Schädlingen.

**17.** Verwendung gemäss Anspruch 16 zur Bekämpfung von Insekten und/oder Spinnentieren.

**18.** Verwendung gemäss Anspruch 16 oder 17 zur Behandlung von Saatgut.

**19.** Verfahren zur Bekämpfung von Schädlingen, dadurch gekennzeichnet, dass man eine Verbindung gemäss Anspruch 1 der Formel I, in freier Form oder in agrochemisch verwendbarer Salzform, oder ein Mittel gemäss Anspruch 13 oder 14 auf die Schädlinge und/oder ihren Lebensraum appliziert.

**20.** Verfahren gemäss Anspruch 19 zur Bekämpfung von Insekten und/oder Spinnentieren.

**21.** Verfahren gemäss Anspruch 19 oder 20 zum Schutz von Saatgut, dadurch gekennzeichnet, dass man das Saatgut und/oder die Saatfurche behandelt.

**22.** Saatgut, behandelt gemäss dem in Anspruch 21 beschriebenen Verfahren.

**Patentansprüche für folgenden Vertragsstaat: ES**

**1.** Verfahren zur Herstellung einer Verbindung der Formel

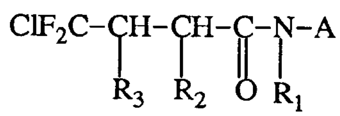

(I),

worin
A einen unsubstituierten oder substituierten, über ein Ringstickstoffatom gebundenen, aromatischen oder nicht-aromatischen, mono- oder bicyclischen, stickstoffhaltigen Heterocyclus oder eine Gruppe $-OR_4$, $-N(R_4)R_5$, $-N(R_5)COR_4$, $-N(R_5)COOR_4$, $-N(R_5)CON(R_4)R_6$ oder $-N(R_5)CSN(R_4)R_6$;
$R_1$ Wasserstoff, $C_1$-$C_6$-Alkyl, $C_1$-$C_6$-Halogenalkyl oder Benzyl;
$R_2$ und $R_3$ unabhängig voneinander Wasserstoff oder $C_1$-$C_4$-Alkyl;
$R_4$ Wasserstoff, eine unsubstituierte oder substituierte $C_1$-$C_{20}$-Alkyl-, $C_3$-$C_{10}$-Alkenyl-, $C_3$-$C_{10}$-Alkinyl-, $C_3$-$C_7$-Cycloalkyl- oder Aryl-Gruppe oder einen unsubstituierten oder substituierten, aromatischen oder nicht-aromatischen, über ein Ringkohlenstoffatom gebundenen, mono- oder bicyclischen, Heterocyclus; und
$R_5$ und $R_6$ unabhängig voneinander Wasserstoff, $C_1$-$C_6$-Alkyl oder Phenyl bedeuten; in freier Form oder in Salzform,
dadurch gekennzeichnet, dass man
a) eine Verbindung der Formel

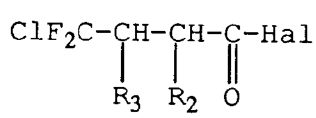

(II),

worin $R_2$ und $R_3$ die für die Formel I angegebenen Bedeutungen haben und Hal für Halogen, vorzugsweise Chlor oder Brom, steht, in Gegenwart einer Base mit einer Verbindung der Formel

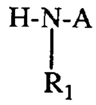

(III),

worin A und $R_1$ die für die Formel I angegebenen Bedeutungen haben, oder einem Salz davon umsetzt oder

b) eine Verbindung der Formel

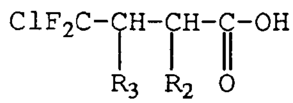

(IV),

worin $R_2$ und $R_3$ die für die Formel I angegebenen Bedeutungen haben, in Gegenwart eines Kondensationsmittels mit einer Verbindung der Formel nI oder einem Salz davon umsetzt oder

c) eine Verbindung der Formel

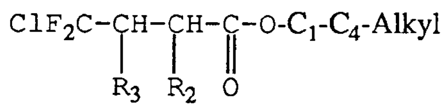

(V),

worin $R_2$ und $R_3$ die für die Formel I angegebenen Bedeutungen haben, mit einer Verbindung der Formel III oder einem Salz davon umsetzt

und jeweils, wenn erwünscht, eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

**2.** Verfahren gemäss Anspruch 1 zur Herstellung einer Verbindung der Formel I in freier Form.

**3.** Verfahren gemäss Anspruch 2 zur Herstellung einer Verbindung der Formel I, worin A für $-OR_4$, $-N(H)R_4$, $-N(CH_3)R_4$, $-N(C_6H_5)R_4$, $-N(H)COR_4$, $-N(CH_3)COR_4$, $-N(H)COOR_4$, $-N(H)CON(H)R_4$, $-N(H)CON(C_6H_5)R_4$, $-N(H)CSN(H)R_4$, Pyrrolidin-1-yl, Piperidin-1 -yl, Perhydroazepin-1-yl, Morpholin-4-yl, 4-Methylpiperazin-1-yl, Triazol-N-yl oder Phthalimid-N-yl steht.

**4.** Verfahren gemäss Anspruch 2 zur Herstellung einer Verbindung der Formel I, worin $R_4$ für $C_1$ -$C_{10}$-Alkyl; $C_3$-$C_7$-Cycloalkyl; $C_3$-$C_{10}$-Alkenyl; $C_3$-$C_{10}$-Alkinyl; Aryl; $C_3$-$C_{10}$-Halogenalkenyl; $C_3$-$C_{10}$-Halogenalkinyl; durch Halogen oder $C_1$-$C_4$-Alkyl substituiertes $C_3$-$C_7$-Cycloalkyl; durch Halogen, $C_1$-$C_4$-Alkyl, $C_1$-$C_4$-Halogenalkyl, $C_1$-$C_{12}$-Alkoxy, $C_1$-$C_4$-Halogenalkoxy, $C_1$-$C_4$-Alkylthio, Nitro, Cyano, Benzoyl, Halogenbenzoyl, Phenoxy, Halogenphenoxy, $C_1$-$C_4$-Alkylphenoxy, $C_1$-$C_4$-Halogenalkylphenoxy, $C_1$-$C_4$-Alkylamino, Di-$C_1$-$C_4$-alkylamino, Anilino, N-$C_1$-$C_4$-Alkylanilino, N-$C_1$-$C_6$-Alkylcarbonylanilino, Phenylthio oder Halogenphenylthio substituiertes Aryl; einen über ein Ringkohlenstoffatom gebundenen, aromatischen oder nicht-aromatischen, mono- oder bicyclischen, Heterocyclus, welcher unsubstituiert oder durch Halogen, $C_1$-$C_4$-Alkyl, Nitro, $C_1$-$C_4$-Halogenalkyl, $C_1$-$C_4$-Halogenalkoxy, $C_1$-$C_4$-Alkoxy, $C_1$-$C_4$-Alkylthio oder Cyclopropyl substituiert sein kann; oder durch Hydroxy, Halogen, Di-$C_1$-$C_4$-alkylamino, $C_1$-$C_4$-Alkoxy, $C_1$-$C_4$-Halogenalkoxy, $C_2$-$C_6$-Alkoxyalkoxy, $C_1$-$C_4$-Halogenalkylthio, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, $C_1$-$C_4$-Alkylcarbonyl, $C_1$-$C_4$-Alkoxycarbonyl, $C_1$-$C_6$-Alkylcarbonyloxy, $C_3$-$C_7$-Cycloalkyl, Aryl, Aryloxy, Arylthio, Arylsulfonyl, Arylsulfinyl, Arylsulfonyloxy, Arylcarbonyl oder Pyridyl substituiertes $C_1$-$C_{20}$-Alkyl, wobei die Aryl- und Pyridylgruppen jeweils durch Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, Nitro, Cyano, Phenoxy, Halogenphenoxy, Phenylthio oder Halogenphenylthio substituiert sein können, steht.

**5.** Verfahren gemäss Anspruch 2 zur Herstellung einer Verbindung der Formel I, worin $R_1$ Wasserstoff ist.

**6.** Verfahren gemäss Anspruch 2 zur Herstellung einer Verbindung der Formel I, worin $R_2$ und $R_3$ Wasserstoff

sind.

7. Verfahren gemäss Anspruch 2 zur Herstellung einer Verbindung der Formel I, worin $R_4$ Wasserstoff; $C_1$-$C_4$-Alkyl; durch Halogen oder $C_1$-$C_4$-Alkoxy substituiertes $C_1$-$C_4$-Alkyl; Phenyl; durch 1 bis 5 Halogenatome oder 1 bis 2 Substituenten, ausgewählt aus der Gruppe, bestehend aus $C_1$-$C_4$-Alkyl, $C_1$-$C_4$-Alkoxy, Cyano, $C_1$-$C_4$-Halogenalkyl und Nitro, substituiertes Phenyl; Benzyl; durch 1 bis 5 Halogenatome oder 1 bis 2 Substituenten, ausgewählt aus der Gruppe, bestehend aus $C_1$-$C_4$-Alkoxy und Nitro, substituiertes Benzyl; Thienyl; oder Furyl bedeutet.

8. Verfahren gemäss Anspruch 2 zur Herstellung einer Verbindung der Formel I,worin $R_1$, $R_2$ und $R_3$ Wasserstoff bedeuten, A Piperidin-1-yl, 4-$C_1$-$C_4$-Alkylpiperazin-1-yl, Phthalimid-N-yl oder eine Gruppe -$OR_4$, -$N(R_4)R_5$, -$N(R_5)COR_4$, -$N(R_5)COOR_4$, -$N(R_5)CON(R_4)R_5$ oder -$N(R_5)CSN(R_4)R_5$ ist, $R_4$ $C_1$-$C_4$-Alkyl; Halogen-$C_1$-$C_4$-alkyl mit 1 bis und mit 3 Halogenatomen; Phenyl; durch 1 bis 5 Halogenatome oder 1 bis 2 Substituenten, ausgewählt aus der Gruppe, bestehend aus $C_1$-$C_4$-Alkoxy und Nitro, substituiertes Phenyl; Benzyl; durch 1 bis 5 Halogenatome oder 1 bis 2 Substituenten, ausgewählt aus der Gruppe, bestehend aus $C_1$-$C_4$-Alkoxy und Nitro, substituiertes Benzyl; Pyridyl; Thienyl; oder Furyl bedeutet, $R_5$ Wasserstoff, $C_1$-$C_4$-Alkyl oder Phenyl ist und $R_5$ Wasserstoff ist.

9. Verfahren gemäss Anspruch 2 zur Herstellung einer Verbindung der Formel I, worin A -$OR_4$, -$N(R_4)R_5$, -$N(R_5)COR_4$ oder -$N(R_5)COOR_4$ ist und $R_5$ Wasserstoff, Methyl oder Phenyl ist.

10. Verfahren gemäss Anspruch 8 zur Herstellung einer Verbindung der Formel I, worin $R_1$, $R_2$ und $R_3$ Wasserstoff bedeuten, A eine Gruppe -$N(R_4)R_5$, -$N(R_5)COR_4$ oder -$N(R_5)COOR_4$ ist, $R_4$ $C_1$-$C_4$-Alkyl, Halogen-$C_1$-$C_4$-alkyl mit 1 bis und mit 3 Halogenatomen, Phenyl, durch 1 bis 5 Halogenatome substituiertes Phenyl oder durch $C_1$-$C_4$-Alkoxy substituiertes Benzyl bedeutet und $R_5$ Wasserstoff oder Phenyl ist.

11. Verfahren gemäss Anspruch 2 zur Herstellung einer Verbindung der Formel I, ausgewählt aus der Gruppe von Verbindungen, bestehend aus
    (a) 4-Chlor-4,4-difluor-N-methoxy-buttersäureamid,
    (b) 4-Chlor-4,4-difluor-N-(äthoxycarbonylamino)-buttersäureamid,
    (c) 4-Chlor-4,4-difluor-N-(phenylamino)-buttersäureamid,
    (d) 4-Chlor-4,4-difluor-N-(phenylcarbonylamino)-buttersäureamid,
    (e) 4-Chlor-4,4-difluor-N-(phenylaminocarbonylamino)-buttersäureamid,
    (f) 4-Chlor-4,4-difluor-N-(N,N-dimethylamino)-buttersäureamid,
    (g) 4-Chlor-4,4-difluor-N-(methylcarbonylamino)-buttersäureamid,
    (h) 4-Chlor-4,4-difluor-N-(fur-2-ylcarbonylamino)-buttersäureamid,
    (i) 4-Chlor-4,4-difluor-N-(4-methoxybenzyloxycarbonylamino)-buttersäureamid,
    (j) 4-Chlor-4,4-difluor-N-(thien-2-ylcarbonylamino)-buttersäureamid,
    (k) 4-Chlor-4,4-difluor-N-(tert.-butoxycarbonylamino)-buttersäureamid und
    (l) 4-Chlor-4,4-difluor-N- (N-methyl-N-phenyl-amino)-buttersäureamid.

12. Schädlingsbekämpfungsmittel, dadurch gekennzeichnet, dass es mindestens eine Verbindung, erhältlich gemäss Anspruch 1, der Formel I, in freier Form oder in agrochemisch verwendbarer Salzform, als Wirkstoff und mindestens einen Hilfsstoff enthält.

13. Mittel gemäss Anspruch 12 zur Bekämpfung von Insekten und/oder Spinnentieren.

14. Verfahren zur Herstellung eines Mittels gemäss Anspruch 12 oder 13, dadurch gekennzeichnet, dass man den Wirkstoff mit dem (den) Hilfsstoff(en) innig vermischt.

15. Verwendung einer Verbindung, erhältlich gemäss Anspruch 1, der Formel I, in freier Form oder in agrochemisch verwendbarer Salzform, oder eines Mittels gemäss Anspruch 12 oder 13 zur Bekämpfung von Schädlingen.

16. Verwendung gemäss Anspruch 15 zur Bekämpfung von Insekten und/oder Spinnentieren.

17. Verwendung gemäss Anspruch 15 oder 16 zur Behandlung von Saatgut.

18. Verwendung einer Verbindung, erhältlich gemäss Anspruch 1, der Formel I, in freier Form oder in agrochemisch verwendbarer Salzform, zur Herstellung eines Mittels gemäss Anspruch 12 oder 13.

**19.** Verfahren zur Bekämpfung von Schädlingen, dadurch gekennzeichnet, dass man eine Verbindung, erhältlich gemäss Anspruch 1, der Formel I, in freier Form oder in agrochemisch verwendbarer Salzform, oder ein Mittel gemäss Anspruch 12 oder 13 auf die Schädlinge und/oder ihren Lebensraum appliziert.

**20.** Verfahren gemäss Anspruch 19 zur Bekämpfung von Insekten und/oder Spinnentieren.

**21.** Verfahren gemäss Anspruch 19 oder 20 zum Schutz von Saatgut, dadurch gekennzeichnet, dass man das Saatgut und/oder die Saatfurche behandelt.

**22.** Saatgut, behandelt gemäss dem in Anspruch 21 beschriebenen Verfahren.